# EUROPÄISCHE PATENTSCHRIFT

(11) **EP 0 720 492 B1**
(45) Veröffentlichungstag und Bekanntmachung des Hinweises auf die Patenterteilung: **07.10.1998**
(21) Anmeldenummer: 95922393.4
(22) Anmeldetag: 03.07.1995
(51) Int. Cl.: A61M 5/315

(54) **VORRICHTUNG ZUM VORSCHUB EINES KOLBENVERSCHIEBEORGANS**
DEVICE FOR ADVANCING A PISTON-MOVING ELEMENT
DISPOSITIF PERMETTANT DE FAIRE AVANCER UN ELEMENT DE DEPLACEMENT DE PISTON

(30) Priorität: 21.07.1994 DE 4425763
(43) Veröffentlichungstag der Anmeldung: 10.07.1996
(73) Patentinhaber: B. Braun Medical AG, 6020 Emmenbrücke (CH)
(72) Erfinder: BERTSCHI, Samuel, CH-6170 Schüpfheim (CH); ZAUGG, Paul, CH-3432 Lützelflüh (CH)
(74) Vertreter: Selting, Günther, Dipl.-Ing.
(86) Internationale Anmeldenummer: CH9500149
(87) Internationale Veröffentlichungsnummer: WO9603170

(56) Entgegenhaltungen:
- EP-A- 0 037 696
- EP-A- 0 627 229
- WO-A-94/06494

## Beschreibung

Die Erfindung betrifft eine Vorrichtung zum Vorschub eines Organs, das zum Vorschub eines Kolbens in einem Zylinder bestimmt ist.

Die genannte Vorrichtung ist vor allem zur Verwendung als Injektionsspritze gedacht, beispielsweise als eine solche, die vom Benützer mitgetragen wird, wie z.B. eine Insulinspritze für Diabetiker. Die bisher bekannten Spritzen wiesen den Nachteil auf, daß das den Spritzenkolben zum Verschieben bringende Mittel, der Betätigungsknopf, bei gefüllter Spritze ein Stück weit vom Spritzenkörper entfernt ist, um diesen Knopf zwecks Ausstoßens der Spritzenflüssigkeit ohne weitere Vorbereitungen gegen den Spritzenkörper drücken zu können. Da nun aber die Benützer wie erwähnt diese Spritzen stets bei sich tragen, besteht die Gefahr einer ungewollten Betätigung, namentlich wenn die Spritze direkt in der Kleidung getragen wird, und damit des Verlustes der für den Benützer lebensnotwendigen Medizin.

Aus WO-A-94/06494, die zur Abgrenzung des Oberbegriffs des Anspruchs 1 herangezogen worden ist, ist eine Spritze mit einer in einem Gehäuse verschiebbaren Hülse bekannt. Die verschiebbare Hülse weist Zähne auf, die in eine ebenfalls mit Zähnen versehene Kolbenstange eingreifen. Die Kolbenstange ist mit einem Kolben verbunden, der in einer mit Injektionsflüssigkeit gefüllten Kartusche angeordnet ist. Durch das Eindrücken der Hülse in das Gehäuse wird die Kolbenstange und der Kolben verschoben, so daß die Spritze betätigt wird. Um die Hülse wieder aus dem Gehäuse herausziehen zu können, weist sowohl die Verzahnung der Hülse als auch die Verzahnung der Kolbenstange Ausnehmungen auf, so daß die Hülse zum Herausziehen gedreht wird, bis sich die Ausnehmungen jeweils mit den Zähnen überdecken. In dieser Stellung kann die Hülse zumindest teilweise aus dem Gehäuse herausgezogen werden, ohne daß die Kolbenstange mitherausgezogen wird. Um die Spritze zu betätigen, muß die herausgezogene Hülse wieder zurückgedreht werden, so daß die Zähne der Hülse wieder über den Zähnen der Kolbenstange angeordnet sind. Die Handhabung dieser Spritze ist relativ aufwendig. Insbesondere Diabetikern, bei denen es sich häufig um ältere Personen handelt, fällt die Handhabung dieser Spritze schwer.

Ferner ist aus WO-A-94/06494 bekannt, die Kolbenstange zusätzlich mit einer Rastverbindung zu versehen, um ein Herausziehen der Kolbenstange zusammen mit der Hülse zu vermeiden. Hierzu weist die Kolbenstange eine Verzahnung auf, in die fest mit dem Gehäuse verbundene Zähne eingreifen. Die Rastverbindung ist so ausgebildet, daß die mit dem Gehäuse verbundenen Zähne beim Eindrücken der Kolbenstange elastisch nachgeben, jedoch ein Herausziehen der Kolbenstange verhindern.

Eine entsprechende Rastverbindung, die lediglich eine Bewegung in eine Richtung zuläßt, ist ferner aus EP-A-0 037 696 bekannt. Hierbei greift in eine auf einem Kolben vorgesehene Längsverzahnung eine Klinke ein. Jeder Zahn der Verzahnung weist eine flache Flanke und eine senkrecht zur Längsachse des Kolbens verlaufende Flanke auf. Die Klinke ist entsprechend komplementär ausgebildet und drehbar gelagert. Somit kann der Kolben in eine Richtung bewegt werden, wobei die Klinke über die flachen Flanken der Verzahnung gleitet. In entgegengesetzter Richtung stößt die Klinke an die senkrechte Flanke und blockiert ein Verschieben des Kolbens.

Aufgabe der Erfindung ist es, bei einfacher Handhabung ein ungewolltes Betätigen der Vorrichtung zu vermeiden.

Eine solche Vorrichtung ist erfindungsgemäß durch die Merkmale des Patentanspruchs 1 gekennzeichnet.

Im folgenden wird die Erfindung anhand eines in den beiliegenden Zeichnungen dargestellten Ausführungsbeispiels einer Injektionsspritze näher erläutert. Sie ist jedoch nicht auf diese Anwendung beschränkt und kann auch auf nichtmedizinalem Gebiet Verwendung finden.

In den Zeichnungen zeigen:
- Fig. 1a-1c: eine dreiteilige auseinandergezogene Darstellung der Vorrichtung in perspektivischer Ansicht,
- Fig. 2: einen Längsschnitt durch die Vorrichtung
und
- Fig. 3-8: Querschnitte entsprechend den Schnitten III-III bis und mit VIII-VIII in Fig. 2.

Anhand der Figuren 1a-1c sollen vorerst diejenigen Teile beschrieben werden, die im zusammengebauten Zustand der Vorrichtung allein noch direkt sichtbar sind. Es sind dies ein Betätigungsmittel 1 der Vorrichtung, das auch als Knopf ausgebildet sein kann und im folgenden als Betätigungsknopf bezeichnet wird, dann eine erste Hülse 2, in welche der Betätigungsknopf in noch zu erläuternder Weise hineinragt, und die, weil sie um ihre Längsachse drehbar ist, als Drehhülse bezeichnet wird; ferner eine zweite Hülse 3, die im folgenden als Führungshülse bezeichnet wird, von der normalerweise nur ein kleiner Abschnitt von größerem Durchmesser sichtbar ist, sowie eine letzte Hülse 4, welche die die abzugebende Flüssigkeit enthaltende Kartusche oder Patrone aufnimmt. Die Drehhülse 2 und die Hülse 4 sowie der erwähnte Abschnitt der Führungshülse 3 bilden den Spritzenkörper oder, allgemeiner ausgedrückt, das Vorrichtungsgehäuse, und weisen daher in ihrem Querschnitt dieselbe äußere Form auf. Die Führungshülse 3 befindet sich etwa je hälftig innerhalb der beiden Hülsen 2 und 4. Das Vorrichtungsgehäuse ist an seinem einen Ende wie erwähnt durch den Betätigungsknopf 1, am andern Ende durch eine auf einen Fortsatz der Hülse 4 aufzusetzende Schutzkappe 5 begrenzt.

Von aussen nicht mehr sichtbar ist eine weitere Hülse, die Vorschubhülse 6. Sie trägt in ihrem Inneren den grössten Teil eines als Zahnstange ausgebildeten Organs 7 und ist selber von einer Schraubenfeder 8 umgeben. Die Feder 8 wird in noch zu erläuternder Weise sowohl auf Druck als auch auf Torsion beansprucht. Das Organ 7 drückt auf noch darzustellende Art bei seiner Verschiebung auf die Kartusche 9 bzw. auf einen in ihr angeordneten Kolben 33 und entleert dadurch die Kartusche.9.

Damit sind die Hauptbestandteile der Vorrichtung aufgezählt. Nun sollen ihre Einzelheiten und ihr Zusammenwirken erläutert werden.

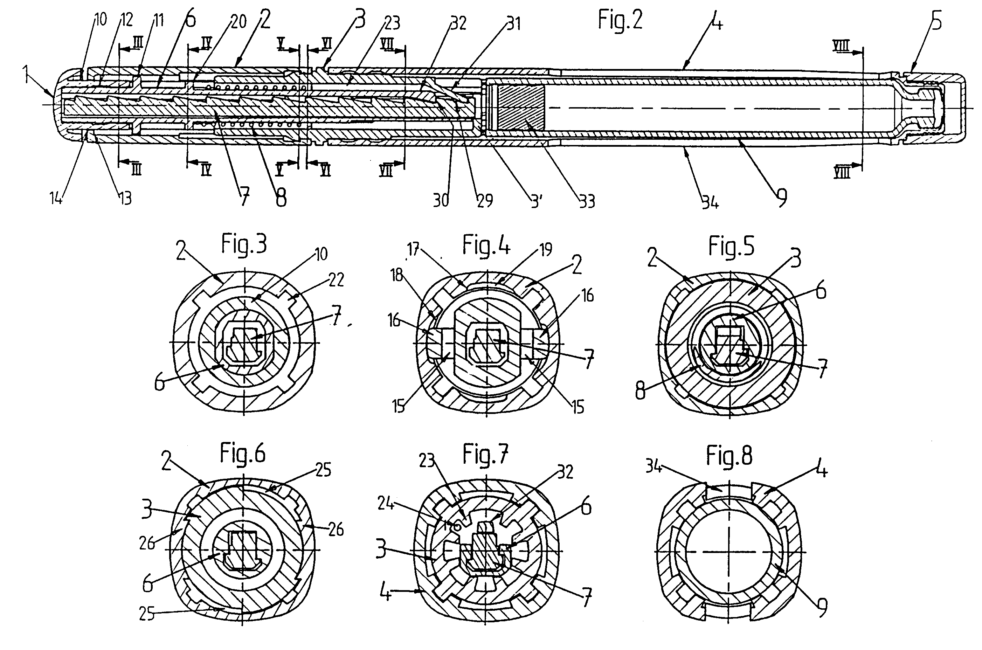

Der Betätigungsknopf 1 weist einen hülsenförmigen Fortsatz 10 auf (Fig. 1a und 2), mittels welchem er das eine Ende der Vorschubhülse 6 umgreift. Dem stirnseitigen Ende dieses Fortsatzes 10 liegt ein Flansch 11 der Vorschubhülse 6 in geringem Abstand gegenüber. Die Vorschubhülse 6 selber setzt sich über diesen Flansch 11 ins Innere des hülsenförmigen Fortsatzes 10 hinein fort. Eine Schnappvorrichtung 12, bestehend aus einer Schnappwulst an der Vorschubhülse 6 und einer Nut im Innern des Fortsatzes 10, verbindet das Betätigungsmittel 1 und die Vorschubhülse 6 nach ihrem Zusammenstecken. Hierbei weitet die Schnappwulst den Fortsatz 10 elastisch etwas auf, bis sie schliseelich in die Nut einschnappt. Dadurch sind das Betätigungsmittel 1 und die Vorschubhülse 6 axial zusammen verschiebbar.

Der soeben erwähnte Flansch 11 der Vorschubhülse 6 dient auch zur Begrenzung dieser Axialverschiebung in der einen Bewegungsrichtung. Die Vorschubhülse 6 befindet sich nämlich mit einem Teil ihrer Länge innerhalb der Drehhülse 2, die an dem bei dem Betätigungsknopf 1 befindlichen Ende einen ringförmigen Abschluss 13 aufweist. Dieser hat eine Öffnung 14, deren Durchmesser nur geringfügig grösser als der des Fortsatzes 10 ist. In der einen Endstellung legt sich somit der Flansch 11 gegen die Innenseite des Abschlusses 13 an. In der andern Endstellung des Betätigungsknopfes 1 stösst die Vorschubhülse 6 gegen die Innenseite einer in Fig. 2 in der Mitte sichtbaren, dort als flanschartige Umbiegung der Führungshülse 3 dargestellten Endwand 3' dieser Hülse an.

Die Vorschubhülse 6 umfasst im weiteren mehrere, vorzugsweise zwei Klauen 15, die auch als Klinken ausgebildet sein können. Eine davon ist aus Fig. 1a ersichtlich. Jede Klaue 15 ist ein längliches, elastisch biegbares Gebilde mit einem Haken 16 an ihrem freien Ende. Durch diese elastische Biegbarkeit kann der Haken radial zur Vorschubhülse bewegt werden, also gegen deren Längsachse bzw. von dieser weg.

Beide Klauen 15 sind auch in Fig. 4 in einer Ruhestellung dargestellt. Ihre Haken 16 sind im Eingriff mit zwei von vier Vertiefungen 17, die in die Innenwand 18 der Drehhülse 2 eingelassen sind. Jede Vertiefung 17, die sich nur über eine geringe Länge in Axialrichtung der Drehhülse 2 erstreckt, ist so angelegt, dass sie bei ihrem einen Ende einen ersten Bereich von maximaler Tiefe und konstantem Radius aufweist. In Umfangsrichtung der Innenwand 18 folgt ein zweiter Bereich, in welchem nun diese Tiefe stetig abnimmt, wie sie in der genannten Figur vor allem aus den beiden nicht benützten Vertiefungen 17 ersichtlich ist, und zwar derart, dass nach einer gewissen Bogenlänge der Innenwand das andere Ende der Vertiefung 17 erreicht ist, an welchem die Tiefe den Wert Null erreicht und die Vertiefung 17 in die Innenwand 18 übergeht. Jede Vertiefung 17 weist eine Seitenwand 19 auf, die in Fig. 4 parallel zur Zeichenebene liegt. Diese Seitenwand 19 dient nun als Anschlagfläche für den entsprechenden Haken 16. Wie ersichtlich, weist wegen der Form der Vertiefung 17 die Seitenwand 19 im ersten Bereich der Vertiefung 17 die grösste Höhe auf, sodass sie den Haken 16 sicher trägt. Die Vorschubhülse 6 und der mit ihr verbundene Betätigungsknopf 1 sind damit in der Ruhestellung gegen axiale Verschiebung arretiert und zwar derart, dass der Betätigungsknopf 1 in der allernächsten Lage zur Drehhülse 2 und damit zum Gehäuse 2, 3, 4 der ganzen Vorrichtung festgehalten ist. Er kann sich also praktisch nicht mehr weiter gegen das Gehäuse hin bewegen, selbst wenn Druck auf ihn ausgeübt wird.

Wie ersichtlich, nimmt mit abnehmender Tiefe des zweiten Bereichs der Vertiefung 17 auch die Auflagefläche der Seitenwand 19 für den Haken 16 ab. Rotiert man nun die Drehhülse 2 um ihre Längsachse in eine Spannstellung und damit um die nicht drehbare Vorschubhülse 6, so bewegen sich die Vertiefungen 17 relativ zu den mit der Vorschubhülse 6 verbundenen Haken 16 derart, dass diese die Klauen 15 wegen der nun stetig abnehmenden Tiefe immer mehr gegen die Längsmittelachse der Vorschubhülse 6 bezw. der Drehhülse 2 hin durchbiegen. Schliesslich in der Spannstellung ist das Uebergangsende der Vertiefungen 17 erreicht. Dort ist keine stützende Seitenwand 19 mehr vorhanden. Damit ist die Arretierung aufgehoben. Die Vorschubhülse 6 kann sich nun axial innerhalb der Drehhülse 2, also an deren zylindrischen Innenwand 18, verschieben, wobei die Haken 16 an dieser Innenwand entlang gleiten. Diese Verschiebung kommt zu Ende, wenn der Flansch 11 der Vorschubhülse 6 durch Anlegen an den Abschluss 13 der Drehhülse 2 gestoppt wird.

Ausgelöst wird diese Bewegung durch die unter Druck stehende Feder 8, welche wie schon erwähnt die Vorschubhülse 6 umgibt und ihrerseits innerhalb der Führungshülse 3 zentriert ist, wo sie auch ihren Sitz hat. Auf diesen wird noch zurückgekommen. Die Feder 8 drückt mit ihrem einen Ende auf einen Flansch 20 an der Aussenseite der Vorschubhülse 6 und schiebt diese nach gelöster Arretierung nach links in Fig. 2. Damit wird der Betätigungsknopf 1 von der Drehhülse 2 axial wegbewegt und gelangt in eine Startposition, in welcher er durch Zurückdrücken in seine Anfangslage auf indirekte und noch zu beschreibende Weise die Kartusche 9 in der als Kartuschenhalter dienenden Hülse 4 leert.

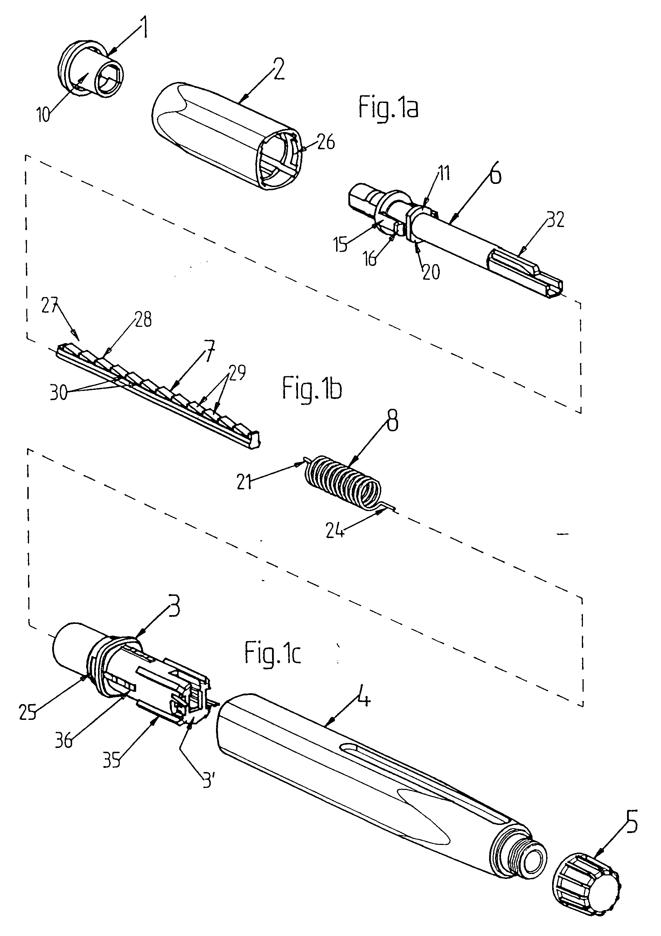

Vorerst muss jedoch noch erwähnt werden, dass die ganze axiale Bewegung des Betätigungsknopfes 1 durch Drehen der Drehhülse 2 aus der Ruhestellung in die Spannstellung ausgelöst würde, was die Arretierung der Klauen 15 bezw. ihrer Haken 16 aufhob. Damit nun die Drehhülse 2 wieder in ihre Ruhe- bzw. Anfangsstellung zurückgedreht wird, ist die Feder 8 an ihrem einen Ende zu einem in Fig. 1b deutlich sichtbaren radial zur Schraubenfeder 8 verlaufenden Fortsatz 21 umgebogen. Dieser greift in eine von mehreren in Längsrichtung verlaufenden und eigentlich aus herstellungstechnischen Gründen angeordneten Nuten 22 (Fig. 3) ein. Verdreht man die Drehhülse 2, wird der Fortsatz 21 durch die Nut 22 mitgenommen und spannt daher die Feder auf Torsion. Durch Loslassen der Drehhülse 2 nach durchgeführter Bewegung des Betätigungsknopfes 1 in seine Startposition wird durch die Torsionsspannung der Feder die Drehhülse 2 automatisch in ihre Ruhestellung zurückgedreht. Wird auch der Betätigungsknopf aus seiner Startposition heraus in seine Anfangslage zurückgeschoben und mit ihm die Vorschubhülse 6, finden deren Klauen 15 die Vertiefungen 17 wieder am alten Ort und rasten mit ihren Haken 16 dort ein. Damit ist die ganze Vorrichtung automatisch wieder verriegelt. Zu beachten ist, dass die Torsionsbeanspruchung der Feder 8 unabhängig von ihrer Druckbeanspruchung erfolgt, welche zur Bewegung der Vorschubhülse 6 ausgenützt wird.

An ihrem andern Ende stützt sich die Feder 8 wie erwähnt in der Führungshülse 3 ab und zwar auf die Stirnseiten von Längsrippen 23 (Fig. 2 und 7), die über die Innenwand der Hülse 3 verteilt angeordnet sind. Ein zweiter Fortsatz 24, der im Gegensatz zum Fortsatz 21 axial verläuft (Fig. 1b und 7), kommt zwischen die Längsrippen 23 zu liegen und verankert so die Feder 8 gegen deren Mitdrehen mit der Drehhülse 2; gleichzeitig kann so die Torsionsvorspannung festgelegt werden. Die Führungshülse 3 weist zwei einander gegenüber liegende Nuten 25 auf, in welche kürzere, von der Drehhülse nach innen vorstehende Nocken 26 eingreifen, wie dies aus Fig. 6 ersichtlich ist. Durch die nicht den ganzen Umfang umfassenden Nuten 25 und die noch kürzeren Nocken 26 werden die Anfangs- und die Endlage der Drehhülse und damit ihr Drehbereich fixiert.

Nunmehr soll, wie weiter oben angeführt, die Leerung der Kartusche 9 durch die Betätigung des Betätigungsknopfes 1 erläutert werden. Dazu dient das Organ 7 im Innern der Vorschubhülse 6. Es weist eine Verzahnung 27 auf, die aus mehreren Zähnen 28 besteht. Jeder Zahn ist aus einer flach ansteigenden Flanke 29 und einer sehr steilen, von der Vertikalen nur wenig abweichenden, also annähernd senkrechten Flanke 30 gebildet. Die in den Fig. 3 - 7 angedeutete treppenförmige Ausbildung der einen Seitenwand des Organs 7 dient mit der im Vergleich zur gegenüberliegenden Seitenwand hochgezogenen unteren Stufe lediglich zum korrekten Einsetzen des Organs 7 in die Vorschubhülse 6, um eine falsche Montage zu verhindern.

Bewegt sich nun die Vorschubhülse in Fig. 2 nach links, müsste sie eigentlich das in ihr angeordnete Organ 7 mitnehmen. Das kann sie aber nicht, denn mit der Verzahnung 27 ist eine erste Klinke 31, die im folgenden auch als Raste bezeichnet ist, (Fig. 2) im Eingriff, die zwar starr an der Führungshülse 3 angebracht ist, jedoch elastisch durchfedern kann. Da sie an der annähernd vertikalen Flanke 30 des betreffenden Zahns anliegt, hält sie das Organ 7 zurück, obwohl eine ebenfalls elastisch federnde zweite Klinke 32 (Fig. 1a und 2), die an der Vorschubhülse 6 angebracht ist und auf der Verzahnung 27 aufliegt, einen gewissen Reibungsdruck auf das Organ 7 ausübt. Wegen der flachen Flanken 29 der Zähne 28 nützt dies aber nichts. Das Organ 7 bleibt stehen, während sich die Vorschubhülse 6 wegbewegt und das Organ 7 teilweise freilegt. Hierbei gleitet die zweite Klinke 32 elastisch federnd über die Verzahnung 27 hinweg, bis sie schliesslich am Ende der Bewegung in einen Zahn einrastet, der vom letzten Zahn, in den die Raste 31 gemäss Fig. 2 vorerst noch einrastet, entfernt ist.

Kommt nun aber die Gegenbewegung durch das Zurückdrücken des Betätigungsknopfes 1, kehren sich die Verhältnisse um. Jetzt ist die Klinke 32 in festem Eingriff mit der Verzahnung 27 und kann daher das Organ 7 bei der Bewegung der Vorschubhülse 6 vor sich herschieben. Organ 7 und Vorschubhülse 6 bewegen sich also mit gleicher Geschwindigkeit. Nun ist es die Raste 31, die dieser Bewegung keinen Widerstand mehr entgegensetzen kann. Dank der flachen Flanken 29 gleitet das Organ 7 unter ihr hinweg, bis sie am Ende der Bewegung in einen andern Zahn einrastet, um bei der nächsten Betätigung der Vorrichtung das Organ 7 an der entgegengesetzten Bewegung zu hindern, wie oben schon erwähnt. Das Organ 7 bewegt sich in die als Kartuschenhalter dienende Hülse 4 hinein und kommt in Kontakt mit einem in der Kartusche eingebauten, mit ihr gelieferten und ihren Inhalt abdichtenden Kolben 33. Diesen schiebt es vor sich her und leert so die Kartusche, was durch ein Sichtfenster 34 in ihr (Fig. 8) beobachtet werden kann.

Zu beachten ist, dass wegen der Raste 31 das Organ 7 sich nur in einer Richtung bewegen kann, während die Vorschubhülse 6 sich hin und her bewegt. Das Organ 7 tritt also bei jeder Betätigung der Vorrichtung mehr und mehr aus der Vorschubhülse 6 heraus. Es bildet also mit der Vorschubhülse 6 zusammen eine zunehmend länger werdende Kolbenstange, die den Kolben 30 schrittweise durch die ganze Kartusche 9 hindurchtreibt, bis diese leer ist.

Durch die Zahnteilung der Zahnstange ist der einzelne Vorschubschritt und damit die Dosierung der Flüssigkeit vorbestimmt und vom Anwender nicht veränderbar, ausser in der Anzahl der Vorschübe.

Auf diese Weise wird eine Spritzwirkung erreicht, ohne dass der Betätigungsknopf 1 schon bei Nichtgebrauch vom Gehäuse 2, 3, 4 weit abstehen muss. Dieser Abstand wird erst unmittelbar vor dem Spritzen herbeigeführt. Vorher und nachher bildet der Betätigungsknopf mit dem Gehäuse ein kompaktes Ganzes, das gegen unbeabsichtigte Betätigung gesichert ist.

Weil die Drehhülse 2 und die Führungshülse 3 nicht in beliebiger Lage zusammengebaut werden können (wegen der Funktion von Vorschubhülse 6 und Organ 7), sind sie im Querschnitt auch nicht kreisrund. Die Montagemöglichkeiten werden dadurch herabgesetzt. Um ein einheitliches Aussehen der ganzen Vorrichtung zu erzielen, erhält vorzugsweise auch die Hülse 4 die gleiche Aussenform wie die Drehhülse 2 bezw. der sichtbare Flansch der Führungshülse 3. Die in Fig. 1c an der Führungshülse 3 sichtbaren Längsrippen 35 und Federrippen 36 sind daher-eine Folge dieser Massnahme und tragen zum gegenseitigen Zentrieren der Hülsen 3 und 4 bei. Der nicht kreisrunde Querschnitt verhindert auch ein ungewolltes Wegrollen der ganzen Vorrichtung.

## Patentansprüche

1. Vorrichtung zum Vorschub eines Organs (7), das zum Vorschub eines Kolbens (33) bestimmt ist, mit einem aus einzelnen Hülsen (2,3,4) bestehenden Gehäuse, und mit einer in dem Gehäuse längsverschiebbar angeordneten, an einem Ende ein Betätigungsmittel (1) aufweisenden, gegen die Wirkung einer Feder (8) vorschiebbaren und dabei den Vorschub des Organs (7) bewirkenden Vorschubhülse (6), in der das Organ (7) längsverschiebbar angeordnet ist, wobei an einer der Hülsen (3,4) des Gehäuses eine Raste (31) für das Organ (7) angebracht ist, die eine Verschiebbarkeit des Organs (7) nur in eine Richtung zuläßt,
**dadurch gekennzeichnet,**
daß eine der Hülsen des Gehäuses als Drehhülse (2) ausgebildet ist, die in der einen Drehrichtung vorgespannt ist und zwischen einer Ruhestellung und einer Spannstellung drehbar ist, daß an der Vorschubhülse (6) mindestens eine Klaue (15) vorgesehen ist, die in der Ruhestellung der Drehhülse (2) in eine Vertiefung (17) der Drehhülse (2) eingreift und dadurch die Vorschubhülse (6) gegen Längsverschiebung in der Drehhülse (2) sichert, und in der Spannstellung außer Eingriff mit der Vertiefung (17) steht, und dadurch die Bewegung der Vorschubhülse (6) unter dem Druck der Feder (8) ohne Mitnahme des Organs (7) in eine den Vorschub des Organs (7) ermöglichende Startposition erlaubt.

2. Vorrichtung nach Anspruch 1, dadurch gekennzeichnet, daß die auf die Vorschubhülse (6) zum axialen Verschieben wirkende Feder (8) zugleich eine Drehfeder zum Vorspannen der Drehhülse (2) bildet.

3. Vorrichtung nach Anspruch 1 oder 2, dadurch gekennzeichnet, daß zum Verschieben der Vorschubhülse (6) in ihre Start position die Feder (8) mit einem Ende an einem als Federsitz ausgebildeten Flansch (20) der Vorschubhülse (6) und mit dem anderen Ende an einer als Führungshülse (3) ausgebildeten Hülse des Gehäuses anliegt.

4. Vorrichtung nach einem der Ansprüche 1-3, dadurch gekennzeichnet, daß die Vertiefung (17) der Drehhülse (2) an einem Ende einen ersten Bereich mit maximaler Tiefe aufweist, in dem die Klaue (15) der Vorschubhülse (6) in Ruhestellung an einer Seitenwand (19) zur Sicherung gegen axiales Verschieben anliegt, und daß an den ersten Bereich ein zweiter Bereich anschließt, in welchem die Tiefe in Umfangsrichtung der Innenwand der Drehhülse (2) stetig bis auf Null abnimmt, so daß die Klaue (15) in der Spannstellung außer Eingriff gelangt.

5. Vorrichtung nach einem der Ansprüche 1-4, dadurch gekennzeichnet, daß das Organ (7) an einer seiner Längsseiten eine Verzahnung (27) aufweist, deren Zähne im Längsquerschnitt eine erste in axialer Richtung flach ansteigende Flanke (29) sowie eine annähernd senkrecht zur Axialrichtung verlaufende zweite Flanke aufweisen und in die Verzahnung (27) eine an der Vorschubhülse (6) angebrachte Klinke (32) eingreift.

6. Vorrichtung nach Anspruch 5, dadurch gekennzeichnet, daß die Raste (31) in die Verzahnung (27) des organs (7) eingreift.

7. Vorrichtung nach einem der Ansprüche 2-6, dadurch gekennzeichnet, daß die Feder (8), um als Drehfeder zu dienen, mit einem Ende (21) in einer Nut (22) in der Drehhülse (2) und mit dem anderen Ende (24) drehfest an der Führungshülse (3) verankert ist, mit welcher auch die Drehhülse (2) über radiale Vorsprünge drehbar, aber axial unverschiebbar, verbunden ist, so daß bei der Relativverdrehung der Drehhülse (2) zur Führungshülse (3) die Feder (8) auf Torsion beansprucht wird, um die Drehhülse (2) wieder in ihre die Klaue (15) der Vorschubhülse (6) arretierende Ruhestellung zurückzudrehen.

8. Vorrichtung nach einem der Ansprüche 1-7, dadurch gekennzeichnet, daß das Betätigungsmittel (1), das in der Ruhelage der Dreh- und Vorschubhülsen (2,6) im wesentlichen unmittelbar an der Drehhülse (2) anliegt, lösbar mit der Vorschubhülse (6) verbunden ist, wobei es mittels eines hülsenartigen Fortsatzes (10) die Vorschubhülse (6) umfaßt und mit dieser über eine Schnappeinrichtung (12) verbunden ist.

9. Vorrichtung nach einem der Ansprüche 1-8 als Injektionsspritze, dadurch gekennzeichnet, daß in die letzte Hülse (4) des Gehäuses eine die Injektionsflüssigkeit sowie einen Kolben (33) in ihrem Innern aufweisende Kartusche (9) einsetzbar ist, wobei der Kolben (33) durch das Organ (7) betätigt wird, um die Kartusche (9) zu entleeren.

## Claims

1. A device for advancing a member (7) intended for advancing a plunger (33), with a housing consisting of separate sleeves (2,3,4) and an advancing sleeve (6) being arranged to be longitudinally displaceable in the housing, comprising an actuation means (1) on an end thereof, being advanceable against the force of a spring (8) and causing the advancement of the member (7), in which sleeve (6) the member (7) is arranged so as to be longitudinally displaceable, with a locking catch (31) for the member (7) being arranged on one of the sleeves (3, 4) of the housing which allows for a displaceability of the member only in one direction,
characterized in
that one of the sleeves of the housing is formed as a rotary sleeve (2) being prestressed in the one rotational direction and being rotatable between a resting position and a stress position, that at least one claw (15) is provided on the advancing sleeve (6) and engages into a recess (17) of the rotary sleeve (2) in the resting position of the rotary sleeve (2), thus securing the advancing sleeve (6) against a longitudinal displacement in the rotary sleeve (2), and is disengaged from the recess (17) in the stress position, thereby allowing to move the advancing sleeve (6) under the pressure of the spring (8), without carrying along the member (7), into a start position of the member (7).

2. The device according to claim 1, characterized in that the spring (8) acting on the advancing sleeve (6) for axial displacement also forms a rotary spring for prestressing the rotary sleeve (2).

3. The device according to claim 1 or 2, characterized in that, for displacing the advancing sleeve (6) into the start position thereof, the spring (8), with one end thereof, rests on a flange (20), formed as a spring seat, of the advancing sleeve (6) and rests, with the other end thereof, on a sleeve formed as a guiding sleeve (3) of the housing.

4. The device according to any one of claims 1-3, characterized in that the recess (17) of the rotary sleeve (2) comprises, on one end thereof, a first area with a maximum depth in which the claw (15) of the advancing sleeve (6) rests, in the resting position, against a lateral wall (19) for securing against an axial displacement and that a second area is adjacent the first area in which the depth steadily decreases to zero in circumferential direction of the interior wall of the rotary sleeve (2) so that the claw (15) disengages in the stress position.

5. The device according to any one of claims 1-4, characterized in that the member (7) has one of its longitudinal sides provided with a toothing (27), the teeth of which comprise, in longitudinal section, a first flank (29) ascending at a small angle in an axial direction as well as a second flank extending approximately vertically to the axial direction and a catch (32) arranged on the advancing sleeve (6) engages into the toothing (27).

6. The device according to claim 5, characterized in that the locking catch (31) engages into the toothing (27) of the member (7).

7. The device according to any one of claims 2-6, characterized in that, to serve as a rotary spring, the spring (8) is anchored with one end (21) in a groove (22) in the rotary sleeve (2) and is non-rotatingly anchored with the other end (24) on the guiding sleeve (3), to which the rotary sleeve (2) is also connected via radial projections so as to be rotatable, but axially non-displaceable, so that the spring (8) is torsionally stressed when the rotary sleeve (2) is rotated relatively to the guiding sleeve (3), in order to turn the rotary sleeve (2) back into its resting position locking the catch (15) of the advancing sleeve (6).

8. The device according to any one of claims 1-7, characterized in that the actuating means (1) substantially resting directly on the end of the rotary sleeve (2) in the resting position of the rotary and advancing sleeves (2,6) is releasably connected to the advancing sleeve (6), the actuating means encompassing the advancing sleeve (6) by means of a sleeve-like extension (10) and being connected thereto via a snap means (12).

9. The device according to any one of claims 1-8 as an injection syringe, characterized in that a cartridge (9) containing the injection liquid as well as a plunger (33) in its interior is insertable into the last sleeve (4) of the housing, said plunger (33) being actuated by the member (7) to empty the cartridge (9).

## Revendications

1. Dispositif pour faire avancer un organe (7) qui est destiné à faire avancer un piston (33), comportant un boîtier constitué par des douilles individuelles (2, 3, 4), et une douille d'avance (6), qui est disposée de manière à être déplaçable longitudinalement dans le boîtier, possède un moyen d'actionnement (1) à une extrémité, peut avancer à l'encontre de l'action d'un ressort (8) et provoque l'avance de l'organe (7), et dans laquelle l;organe (7) est disposé de manière à être déplaçable longitudinalement, sur l'une des douilles (3, 4) du boîtier étant monté un cliquet (31) pour l'organe (7), qui permet une mobilité de l'organe (7) uniquement dans une direction,
caractérisé en ce
que l'une des douilles du boîtier est agencée sous la forme d'une douille rotative (2), qui est précontrainte dans un sens de rotation et peut tourner entre une position de repos et une position de serrage, que sur la douille d'avance (6) est prévue au moins une griffe (15), qui, lorsque la douille rotative (2) est dans la position de repos, s'engage dans un renfoncement (17) de la douille rotative (2) et bloque de ce fait la douille d'avance (6) contre un déplacement longitudinal dans la douille rotative (2), et qui, lorsque la douille dans la poisition de serrage, est dégagée du renfoncement (17), et permet de ce fait le déplacement de la douille d'avance (6) sous la pression du ressort (8), sans entraînement de l'organe (7), dans une position de départ permettant l'avance de l'organe (7).

2. Dispositif selon la revendication 1, caractérisé en ce que le ressort (8), qui agit sur la douille d'avance (6) pour réaliser le déplacement axial, forme simultanément un ressort de torsion servant à précontraindre la douille rotative (2).

3. Dispositif selon la revendication 1 ou 2, caractérisé en ce que pour le déplacement de la douille d'avance (6) pour l'amener dans sa position de départ, le ressort (8) s'applique, par une extrémité, contre une bride (20), qui est agencée sous la forme d'un siège pour ressort, de la douille d'avance (6) et s'applique, par l'autre extremité, contre une douille du boîtier, qui est agencée sous la forme d'une douille de guidage (3).

4. Dispositif selon l'une des revendications 1-3, caractérisé en ce que le renfoncement (17) de la douille rotative (2) possède, sur une extrémité, une première partie ayant une profondue maximale, dans laquelle la griffe (15) de la douille d'avance (6) s'applique, dans sa position de repos, contre une paroi latérale (19) pour s'opposer à un déplacement axial, et qu'à la première zone se raccorde une seconde zone, dans laquelle la profondue diminue continument jusqu'à zéro dans la direction circonférentielle de la paroi intérieure de la douille rotative (2), de sorte que dans la position de serrage, la griffe (15) se dégage.

5. Dispositif selon l'une des revendications 1-4, caractérisé en ce que l'organe (7) possède, sur l'un de ses côtés longitudinaux, une denture (27) dont les dents possèdent, en coupe longitudinale, un premier flanc (29), qui remonte à à plat dans la direction axiale, ainsi qu'un deuxième flanc approximativement perpendiculaire à la direction axiale, et qu'un cliquet (32) installé sur la douille d'avance (6) s'engage dans la denture (27).

6. Dispositif selon la revendication 5, caractérisé en ce que le cliquet (31) s'engage dans la denture (27) de l'organe (7).

7. Dispositif selon l'une des revendications 2-6, caractérisé en ce que, pour servir de ressort de torsion, le ressort (8) est ancré par une extrémité (21) dans une rainure (22) formée dans la douille rotative (2), et par son autre extrémité (24), avec blocage en rotation sur la douille de guidage (3), avec laquelle la douille rotative (2) est également reliée de manière à pouvoir tourner, mais en étant immobile axialement, par l'intermédiaire d'appendices saillants radiaux, de sorte que, lors de la rotation relative de la douille rotative (2) par rapport à la douille de guidage (3), le ressort (8) est sollicité en torsion, de manière à faire pivoter en sens inverse la douille rotative (2) pour l'amener à nouveau dans sa position de repos dans laquelle elle bloque la griffe (15) de la douille d'avance (6).

8. Dispositif selon l'une des revendication 1-7, caractérisé en ce que le moyen d'actionnement (1), qui, lorsque la douille rotative et la douille d'avance (2, 6) sont dans la position de repos, s'applique essentiellement directement contre la douille rotative (2), est reliée de façon amovible à la douille d'avance (6), le moyen d'actionnement enveloppant, au moyen d'un prolongement en forme de douille (10), la douille d'avance (6) et étant relié à cette dernière par l'intermédiaire d'un dispositif à encliquetage (12).

9. Dispositif selon l'une des revendications 1-8, en tant que seringue d'injection, caractérisé en ce qu'une cartouche (9), qui comporte un liquide d'injection ainsi qu'un piston (33) en son intérieur, peut être insérée dans la dernière douille (4) du boîtier, le pistion (33) étant actionné par l'organe (7) de manière à vider la cartouche (9).
